# EUROPÄISCHE PATENTSCHRIFT

(11) **EP 2 677 024 B1**
(45) Veröffentlichungstag und Bekanntmachung des Hinweises auf die Patenterteilung: **20.11.2019**
(21) Anmeldenummer: 13169195.8
(22) Anmeldetag: 24.05.2013
(51) Int. Cl.: C12M 1/00, C12M 1/26

(54) **Vorrichtung zum Separieren von adulten Stammzellen**
Device for separating adult stem cells
Dispositif de séparation de cellules souches adultes

(30) Priorität: 22.06.2012 DE 102012210653; 19.07.2012 US 201261673363 P
(43) Veröffentlichungstag der Anmeldung: 25.12.2013
(73) Patentinhaber: Human Med AG, 19061 Schwerin (DE)
(72) Erfinder: Winkler, Konrad-Wenzel, 19417 Warin (DE); Matthiesen, Inge, 23919 Behlendorf (DE)
(74) Vertreter: Gulde & Partner

(56) Entgegenhaltungen:
- EP-A1- 2 371 943
- EP-A1- 2 389 881
- WO-A1-2010/069080
- WO-A1-2011/079217
- WO-A1-2013/030761
- WO-A2-2006/127007
- WO-A2-2011/145075
- DE-A1-102008 027 486
- DE-A1-102011 080 218
- US-A- 5 372 945
- US-A1- 2010 112 696
- US-A1- 2010 285 588

## Beschreibung

Die vorliegende Erfindung betrifft eine Vorrichtung zum Separieren von adulten Stammzellen aus einem einer biologischen Struktur entnommenen Fettgewebe.

Es ist bekannt, Fettgewebe aus biologischen Strukturen, beispielsweise in der Humanmedizin, zu gewinnen. Fettgewebe wird bekanntermaßen durch geeignete Absaugeinrichtungen abgesaugt und in so genannten Fettgewebezellensammlern gesammelt.

Bekannt ist, dass das so gewonnene Fettgewebe neben Fettzellen auch Stammzellen umfasst. Stammzellen sind Körperzellen, die sich in verschiedene Zelltypen oder Gewebe ausdifferenzieren können. Bei den im Fettgewebe vorhandenen Stammzellen handelt es sich um so genannte adulte Stammzellen.

Eine Gewinnung derartiger adulter Stammzellen aus Fettgewebe ist beispielsweise aus WO 2010/073808 A1 bekannt. Dabei wird das Fettgewebe mit einem Enzym enthaltenden Fluid versetzt und das Gemisch anschließend in einem Behälter zentrifugiert. Hierdurch erfolgt ein Lösen der Stammzellen von den Fettzellen, so dass diese gesondert gesammelt werden können.

Das Separieren kann mittels mechanischer Einrichtungen, z. B. einem Fluidstrahl, wie dies beispielsweise aus DE 10 2011 080 218 A1 bekannt ist, erfolgen.

Aus WO 2006/127007 A2 ist eine Vorrichtung und ein Verfahren zum Separieren von Stammzellen bekannt. Die Vorrichtung umfasst mehrere räumlich getrennt voneinander angeordnete Kammern, die über zusätzliche Leitungen miteinander verbunden sind.

Die WO 2013/030761 A1 offenbart weiterhin ein System zum Separieren von Stammzellen aus einem einer biologischen Struktur entnommenen Fettgewebe. Dabei ist eine Prozesseinheit vorgesehen, in der das Fettgewebe gewaschen wird. Hierdurch erfolgt eine Trennung der Fettzellen von den übrigen Zellen.

US 2010/112696 A1 offenbart eine weitere Vorrichtung zur Trennung von Gewebe und von in dem Gewebe enthaltenen Zellen.

Aus US 5 372 945 A ist des Weiteren eine Vorrichtung zur Aufbereitung von Gewebe bekannt, mit der ein endotheles Zellprodukt herstellbar ist.

Ferner geht aus der US 2010/285588 A1 eine Vorrichtung hervor, mit der Zellen aus einer Gewebeprobe separierbar sind. Die Vorrichtung weist eine Reihe miteinander verbundener Kammern aus, wobei eine Vorfilterstufe als Radialfilter ausgebildet sein kann.

Die vorliegende Erfindung betrifft eine Vorrichtung zum Separieren von Zellen, vorzugsweise adulten Stammzellen, aus einem einer biologischen Struktur, vorzugsweise menschlichem oder tierischem Gewebe, entnommenen Gewebe, vorzugsweise Fettgewebe. Die betreffende Vorrichtung weist einen Behälter auf, auch als Stammzellenkollektor zu bezeichnen, der Aufnahme eines Stoffgemischs dient, wobei das Stoffgemisch das Gewebe, vorzugsweise Fettgewebe, und Zellen, vorzugsweise die adulten Stammzellen, umfasst. Auch kann das Stoffgemisch Lipoaspirat oder Knochenmark enthalten. Die betreffende Vorrichtung ist mit wenigstens einer Spülmittelzuführeinrichtung versehen, die ausgebildet ist, ein Fluid in den Behälter einzuleiten. Vorzugsweise umfasst das Fluid ein Enzym. Vorteilhafterweise kann dadurch eine mechanische Trennung/Separation mittels eines enzymatischen Verdaus unterstützt werden. Ebenso weist die betreffende Vorrichtung eine Stoffgemischzuführeinrichtung auf, die ausgebildet ist, das Stoffgemisch in den Behälter einzuleiten. Auch kann die Spülmittelzuführeinrichtung und die Stoffgemischzuführeinrichtung eine und dieselbe Öffnung im Behälter sein. Vorzugsweise sind diese Zuführeinrichtungen jedoch Öffnungen im Behälter, welche voneinander verschieden und/oder voneinander beabstandet sind. Auch weist die betreffende Vorrichtung eine Stammzellenentnahmeeinrichtung auf, die ausgebildet ist, die separierten Zellen, vorzugsweise die separierten Stammzellen, dem Behälter zu entnehmen. Vorteilhafterweise ist es möglich, dass die Stammzellenentnahmeeinrichtung ein Anschlusselement zum Anschluss einer Spritze aufweist, mit der die Stammzellen abgesaugt werden können. Weiterhin weist die betreffende Vorrichtung wenigstens eine Spülmittelabführeinrichtung auf, die ausgebildet ist, ein um adulte Stammzellen reduziertes Stoffgemisch aus dem Behälter abzuführen. Bevorzugterweise wird das Spülmittel bei der Abführung aus dem Behälter entfernt.

Gegenstand der vorliegenden Erfindung ist eine Vorrichtung zum Separieren von adulten Stammzellen aus einem einer biologischen Struktur entnommenen Fettgewebe, aufweisend:
- einen Behälter zur Aufnahme eines Stoffgemischs, welches das Fettgewebe und die adulten Stammzellen umfasst und der wenigstens drei Kammern aufweist;
- wenigstens eine Spülmittelzuführeinrichtung, die ausgebildet ist, ein Fluid in den Behälter einzuleiten;
- eine Stoffgemischzuführeinrichtung, die ausgebildet ist, das Stoffgemisch in den Behälter einzuleiten;
- eine Stammzellenentnahmeeinrichtung, die ausgebildet ist, die separierten Stammzellen dem Behälter zu entnehmen;
- wenigstens eine Spülmittelabführeinrichtung, die ausgebildet ist, ein um adulte Stammzellen reduziertes Stoffgemisch aus dem Behälter abzuführen und
- wenigstens zwei spezifisch durchlässige Membranen, die für jeweils nur einen spezifischen Teil des jeweiligen Stoffgemischs durchlässig sind,
   wobei
- die wenigstens drei Kammern wenigstens eine erste Kammer, eine dritte Kammer und eine vierte Kammer umfassen,
- von denen die erste Kammer strömungstechnisch entweder direkt mit der dritten Kammer oder mit einer zweiten Kammer verbunden ist, wobei im letzten Fall die zweite Kammer wiederum strömungstechnisch direkt mit der dritten Kammer verbunden ist und
- von denen die dritte Kammer strömungstechnisch direkt mit der vierten Kammer verbunden ist, wobei
- die erste Kammer von der dritten Kammer durch wenigstens eine Membran getrennt ist, die für die adulten Stammzellen durchlässig ist und
- die dritte Kammer von der vierten Kammer durch eine Membran getrennt ist, die undurchlässig für die adulten Stammzellen ist und weiterhin
- die erste Kammer mit der Stoffgemischzuführeinrichtung und der Spülmittelzuführeinrichtung verbunden ist,
- die dritte Kammer mit der Stammzellenentnahmeeinrichtung verbunden ist und
- die vierte Kammer mit der Spülmittelabführeinrichtung verbunden ist.

Erfindungsgemäß ist vorgesehen, dass die Membran, die die dritte Kammer von der vierten Kammer trennt, als Radialfilter ausgebildete ist und die dritte Kammer seitlich umgibt.

Mit anderen Worten weist die räumliche Erstreckung der wenigstens einen Membran die Form eines Zylindermantels, auch als Radialfilter bezeichnet, auf.

Eine Porengröße der fünften Membran, also des Radialfilters, beziehungsweise eine Lochgröße des fünften Siebs kann zwischen 0,1 µm und 10 µm, bevorzugter zwischen 0,3 µm und 5 µm und noch bevorzugter zwischen 0,5 µm und 3 µm betragen.

Die letzte Filterstufe ist wie oben gesagt als Radialfilterstufe ausgebildet. Durch den senkrecht stehenden Radialfilter ergibt sich vorteilhafterweise eine vergrößerte Filterfläche in der letzten Ultrafilterstufe. Dabei können die Stammzellen nach unten absinken, direkt zur Sammelstelle (Reservoir), ohne auf dem Sieb (ohne auf dem Radialfilter) aufzuliegen. Hierdurch wird der Gefahr entgegengewirkt, dass die Stammzellen sich an beziehungsweise auf dem Sieb (auf dem Radialfilter) festsetzen.

Nach der letzten Filterstufe kann die Stammzellsuspension über eine spezielle Ableitung aus dem System herausgeführt werden. Hierbei ist die Porengröße des Radialfilters so eingestellt, dass die Stammzellen innerhalb des Radialfilters bleiben und die Spülflüssigkeit mit den auszuspülenden Bestandteilen durch den Radialfilter hindurchtritt und über den Spüllösungsausgang (die Spülmittelabführeinrichtung), in welchem (in welcher) vorzugsweise Vakuum anliegt, abgesaugt wird. Die Stammzellen bleiben innerhalb des Radialfilters und sinken aufgrund der Schwerkraft nach unten in das Reservoir, von wo sie dann gesondert entnommen werden können.

Vorteilhafterweise ist es möglich, dass die Spülmittelzuführeinrichtung, die Stoffgemischzuführeinrichtung, die Stammzellenentnahmeeinrichtung und/oder die Spülmittelabführeinrichtung jeweils ein Ventil aufweisen, welches ausgebildet ist, zu einer bestimmbaren Zeit eine bestimmbare Menge durchzulassen.

Durch einen optionalen zusätzlichen Zulaufanschluss für eine Spüllösung, also eine Spülmittelzuführeinrichtung, und einen optionalen Ablaufanschluss für die Spüllösung, also eine Spülmittelabführeinrichtung, ist vorteilhafterweise ein optimales Waschen der Stammzellsuspension, zur Abtrennung von Enzymen, Substanzen, Proteinen, Blutbestandteilen oder dergleichen, möglich.

Vorzugsweise weist jede Kammer jeweils eine Spülmittelzuführeinrichtung und eine Spülmittelabführeinrichtung auf, damit vorteilhafterweise innerhalb jeder Kammer ein separates Waschen und Austragen möglich ist. Auch ist es bevorzugt, dass in einer Kammer die Spülmittelzuführeinrichtung und die Spülmittelabführeinrichtung in einer Öffnung zusammentreffen.

Ebenso weist die Vorrichtung wenigstens zwei spezifisch durchlässige Membranen, auch als Filter zu bezeichnen, auf, die für jeweils nur einen spezifischen Teil des jeweiligen Stoffgemischs durchlässig ist. Die wenigstens zwei Membranen können auch als Filteranordnung bezeichnet werden. Eine Anordnung von mehreren Membranen kann auch als Filterebenen oder als Filtergruppe bezeichnet werden. Die Filteranordnung umfasst wenigstens eine Filterebene oder Filtergruppe, vorzugsweise mehrere Filterebenen oder Filtergruppen. Die gegebenenfalls einzelnen aufeinander folgenden Filterebenen oder Filtergruppen können hierbei eine unterschiedliche Porengröße aufweisen, so dass lediglich die Stammzellen jeweils durchgelassen werden und gegebenenfalls größere Partikel, größer als die Stammzellen, stufenweise zurückbleiben, also herausgefiltert werden.

Die Membran kann auch ein Sieb sein. Anstatt der Porengröße weist das Sieb eine Lochgröße auf, welche die maximale Größe eines das Sieb passierbaren Partikels angibt. Eine Größenangabe der Porengröße oder der Lochgröße bezieht sich auf einen äquivalenten Durchmesser, welchen ein Partikel aufweist, der gerade noch durch das Loch des Siebs beziehungsweise durch die Pore der Membran passt.

Eine Porengröße der ersten Membran beziehungsweise eine Lochgröße des ersten Siebs kann zwischen 70 µm und 150 µm, bevorzugter zwischen 80 µm und 130 µm und noch bevorzugter zwischen 90 µm und 110 µm betragen.

Eine Porengröße der zweiten Membran beziehungsweise eine Lochgröße des zweiten Siebs kann zwischen 20 µm und 65 µm, bevorzugter zwischen 30 µm und 50 µm und noch bevorzugter zwischen 35 µm und 45 µm betragen.

Eine Porengröße der dritten Membran beziehungsweise eine Lochgröße des dritten Siebs kann zwischen 5 µm und 65 µm, bevorzugter zwischen 10µm und 40 µm und noch bevorzugter zwischen 15 µm und 25 µm betragen.

Eine Porengröße der vierten Membran beziehungsweise eine Lochgröße des vierten Siebs kann zwischen 1 µm und 30 µm, bevorzugter zwischen 2,5 µm und 22 µm und noch bevorzugter zwischen 5 µm und 15 µm betragen.

Der Behälter weist wenigstens drei Kammern, auch als Filterstufen zu bezeichnen, auf, die durch wenigstens zwei Membranen voneinander getrennt sind, wobei die Stoffgemischzuführeinrichtung und die Stammzellenentnahmeeinrichtung durch wenigstens zwei Membranen voneinander getrennt sind. Eine solche Kammer kann auch als Filterebene bezeichnet werden. Vorzugsweise ist in jeder Filterebene ein Austausch der Spülflüssigkeit und somit ein Austrag der herausgefilterten größeren Partikel möglich. Vorzugsweise kann mittels der Spülmittelzuführeinrichtung jeder Filterebene gegebenenfalls auch gleichzeitig neue Spülflüssigkeit zugeführt werden. Dies erfolgt beispielsweise durch Anlegen entsprechender Unterdrücke an die entsprechende Kammer.

Die wenigstens drei Kammern sind strömungstechnisch direkt miteinander verbunden. Dadurch kann das Stoffgemisch von einer zur nächsten Filterstufe gelangen.

Die Filter in jeder Filterebene sind vorzugsweise öffnen- beziehungsweise schließbar. Hierdurch kann der Zeitpunkt des Beginns der Filterung eingestellt werden. Somit lassen sich in jeder Filterstufe die Abwaschungen der Stammzellen von den dann in den Ebenen der Filter noch vorhandenen Gewebezellen beziehungsweise Gewebezellenresten optimieren.

In einer bevorzugten Ausgestaltung der Erfindung ist vorgesehen, dass die wenigstens drei Kammern wenigstens ein Druckausgleichventil aufweisen, welches ausgebildet ist, einen Druck im Inneren des Behälters gegenüber einem Druck außerhalb des Behälters auszugleichen, und/oder welches weiterhin ausgebildet ist, wiederholbar geöffnet und geschlossen zu werden.

Vorteilhaft daran ist, dass eine Einwirkzeit von beispielsweise Enzymen oder Spüllösungen für jede Kammer separat bestimmt werden kann.

In weiterer bevorzugter Ausgestaltung der Erfindung ist vorgesehen, dass wenigstens eine von den wenigstens drei Kammern ein mechanisch aktives Element aufweist, welches ausgebildet ist, das Fluid mit dem Stoffgemisch zu vermischen.

Vorteilhafterweise wird dadurch die Zeit zum Gewinnen der Stammzellen verkürzt.

Darüber hinaus ist in bevorzugter Ausgestaltung der Erfindung vorgesehen, dass das aktive Element vom Fluid durchströmbar und weiterhin ausgebildet ist, dass ein dynamischer Unterdruck infolge einer Fließgeschwindigkeit des Fluids das Stoffgemisch an das Fluid ansaugt und das Stoffgemisch in Richtung der Fließgeschwindigkeit des Fluids beschleunigt wird. Als Vorteil ergibt sich, dass die Trennung der Stammzellen von den Fettgewebezellen mittels eines Fluidstrahls erfolgt.

Vorzugsweise wird der Fluidstrahl mit verschiedenen Drücken als Flächenstrahl unter Luftabschluss in das Gewebe-Stammzellen-Gemisch eingeleitet. Auch kann die Ableitung der über den Fluidstrahl zugeführten Lösung durch ein Rückschlagventil ermöglicht werden.

Vorzugsweise weist das aktive Element eine Venturi-Düse auf, welche eine Düsenkapillare und eine in Strahlrichtung an der Düsenkapillare befindliche Austrittsöffnung aufweist. Dabei kann das Fluid in die Kammer strömen, das Stoffgemisch infolge des dynamischen Unterdrucks ansaugen und vorteilhafterweise eine mechanische Trennung und/oder eine enzymatische Trennung verbessern.

Das aktive Element kann ein Prallelement aufweisen, welches derart angeordnet ist, dass das beschleunigte Stoffgemisch mit dem strömenden Fluid auf das Prallelement trifft.

Hierdurch ist vorteilhaft eine mechanische Trennung und eine verbesserte Durchmischung mit dem Spülmittel und/oder eine verbesserte Benetzung mit dem Enzym möglich.

In einer besonders bevorzugten Variante weist das aktive Element ein Rührelement, vorzugsweise ein Rührpendel, auf, welches ausgebildet ist, eine quirlartige Drehbewegung und/oder eine schaukelartige Pendelbewegung auszuführen. Ein Antrieb des Rührelementes kann pneumatisch, entweder durch Überdruck und/oder durch Unterdruck, erfolgen. Vorteilhafterweise unterstützt das Rührelement die mechanische Trennung/Separation. Die dann von den Gewebezellen gelösten Stammzellen können durch eine Flüssigkeit, die gegebenenfalls mit Enzymen versetzt ist, gewaschen und über eine Filteranordnung geführt werden.

Vorzugsweise weist das aktive Element einen ortsveränderlichen Stempelkolben auf, der ausgebildet ist, infolge seiner räumlichen Ausdehnung das Fluid mit dem Stoffgemisch zu verdrängen und wiederholbar seinen Ort der Verdrängung zu verändern. Vorteilhafterweise führt die dadurch entstehende Strömungsbewegung innerhalb des Stoffgemischs zu einer verbesserten Durchmischung.

Auch ist es möglich, dass der Stempelkolben pneumatisch, insbesondere mittels Vakuum, zur Oszillation antreibbar ist.

Gemäß einer weiteren vorteilhaften Ausgestaltung der Erfindung weist der Behälter einen Vibrator auf, welcher ausgebildet ist, das Fluid mit dem Stoffgemisch in Schwingung zu versetzen. Dabei werden Teile der Vorrichtung oder dabei wird die Vorrichtung einer Vibration unterzogen, so dass sich hierdurch die Trennung der Stammzellen von den Gewebezellen beziehungsweise von den Gewebezellenresten verbessern lässt. Zusätzlich wird durch den Vibrator das Absinken der Stammzellen im Stoffgemisch beschleunigt. Der Vibrator kann mit mindestens einer Membran schwingungstechnisch verbunden, beispielsweise unterhalb der Membran angeordnet, sein. Der Vibrator kann einen Schallwandler wie beispielsweise ein Piezo-Element, eine polarisierte Folie oder eine Schwingspule aufweisen.

Auch ist es denkbar, einen Vibrator über mindestens einer Membran anzuordnen oder den Vibrator über die Membran führbar anzuordnen. Als Vorteil ergibt sich, dass durch die Schwingung ein Zusetzen der Membran verringert wird.

Auch ist es möglich, dass die Verbindung zwischen den direkt miteinander verbundenen Kammern ein Ventil aufweist, welches ausgebildet ist, wiederholbar geöffnet und geschlossen zu werden. Dadurch kann das Stoffgemisch zu einem festlegbaren Zeitpunkt in einer festlegbaren Menge von einer zur nächsten Filterstufe gelangen. Dies kann auch als ein Öffnen und Schließen des Filters bezeichnet werden, da unterhalb mindestens eines Filters ein Ventil angeordnet ist.

Vorzugsweise weist der Behälter ein Temperierelement auf, welches ausgebildet ist, das Fluid und/oder das Stoffgemisch zu kühlen und/oder zu erwärmen. Vorzugsweise ist das Temperierelement in einem Mantel des Behälters beziehungsweise im Mantel der jeweiligen Filterstufe angeordnet. Dabei kann das Temperierelement ein Peltier-Element aufweisen.

Bevorzugt ist es, dass wenigstens eine Membran eine elektrostatische Polarisierung aufweist. Vorzugsweise umfasst die Membran, mindestens teilweise, mindestens ein Elektret oder besteht vollständig aus mindestens einem Elektret. Als Elektrete möglich sind beispielsweise Polymere wie Polytetrafluorethylen, Polytetrafluorethylenpropylen, Polypropylen, Polyethylenterephthalat, Polyvinylidenfluorid, Siliciumdioxid oder Siliciumnitrid.

Als Vorteil ergibt sich, dass infolge einer gleichartigen elektrostatischen Polarisierung eine Abstoßungskraft zwischen den gleichen Polen auftritt und somit das zu filternde Stoffgemisch als auch die den Filter beziehungsweise die Membran durchlaufenden Partikel nicht an der Membran beziehungsweise am Filter anhaften.

Ebenfalls bevorzugt ist es, dass die Kammer ein geschlossenes Volumenelement, auch als Volumenkörper zu bezeichnen, aufweist, dessen Volumen eine Größe von vorzugsweise mindestens 40 %, bevorzugter mindestens 50 %, weiterhin bevorzugt mindestens 60 %, ebenfalls bevorzugt mindestens 70 %, bevorzugter mindestens 80 % und noch bevorzugter mindestens 90 % eines Volumens der Kammer aufweist. Vorzugsweise wird das Volumenelement durch entsprechende Vibrationseinrichtungen, beispielsweise durch den Vibrator, in Schwingung versetzt. Vorteilhaft dabei sind die dadurch erzielbare Durchmischung und Trennung der Stammzellen von den Fettgewebezellen sowie eine dadurch erzielbare Reinigung des Siebes.

Damit die fünfte Membran durch eine optional wechselnde Saug- und Druckspülung über die Spülmittelabführeinrichtung nicht beschädigt wird, ist die fünfte Membran vorzugsweise durch ein Filtergehäuse in Saugrichtung abgestützt.

Bei der optionalen Druckumkehr/Druckspülung kann das Spaltmaß zwischen der fünften Membran und dem Volumenelement derart bemessen sein, dass sich die fünfte Membran am Volumenelement abstützt.

Vorzugsweise weist das Filtergehäuse und das geschlossene Volumenelement radiale Schlitze auf. Die Schlitze sind also nicht durchgehende Aufweitungen, für eine optimale Durchlässigkeit der fünften Membran. Die Schlitze werden vorzugsweise dann wirksam, wenn die fünfte Membran bei der optionalen Saug-/Druckspülung abgestützt wird.

Vorteilhafterweise verringert der Volumenkörper das Restvolumen der Lösung, in welchem sich die gewonnenen Stammzellen befinden, wodurch auf ein Zentrifugieren verzichtet werden kann.

Vorzugsweise ist der Behälter in Einzelteile zerlegbar, damit die Einzelteile besser gereinigt werden können. Die Einzelteile können dabei Glaszylinder, die einzelnen Kammern, Membranen, den Radialfilter, die Ventile, den Vibrator, das Temperierelement und/oder das aktive Element umfassen.

Die Erfindung wird nachfolgend anhand der zugehörigen Zeichnungen näher erläutert. Es zeigen:
- Figur 1: eine schematische Darstellung einer Vorrichtung zum Separieren von Stammzellen gemäß einer ersten Ausführungsvariante;
- Figur 2: eine schematische Darstellung einer Vorrichtung zum Separieren von Stammzellen gemäß einer zweiten Ausführungsvariante und
- Figur 3: eine schematische Darstellung einer vorteilhaften Ausführungsvariante einer Venturi-Düse.

Für ein Element, welches in Funktion und Aufbau einem Element aus einer davor genannten Figur entspricht, wird dasselbe Bezugszeichen verwendet.

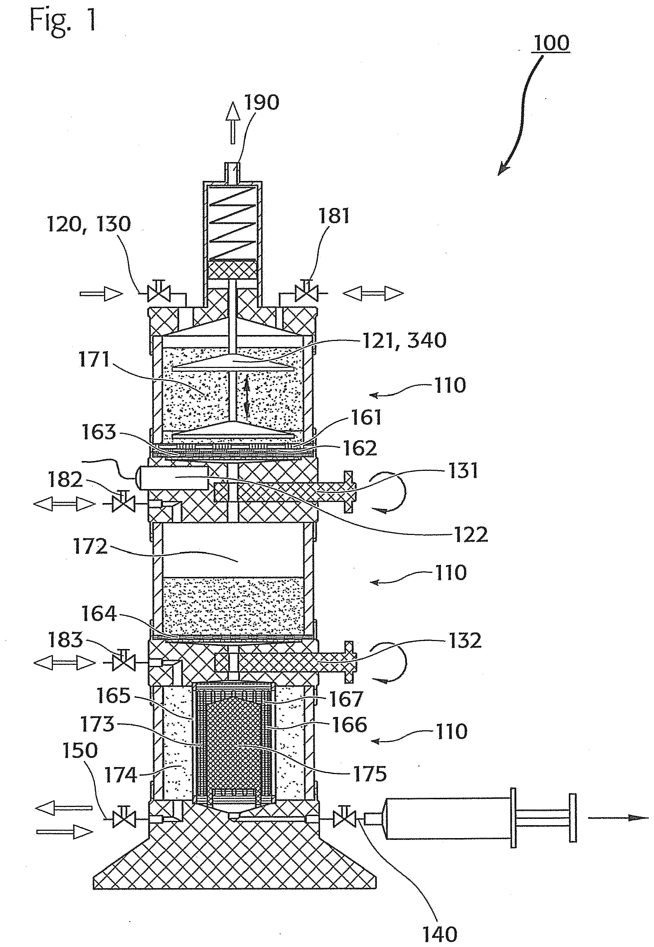

Figur 1 zeigt eine zweidimensionale schematische Darstellung einer insgesamt mit 100 bezeichneten Vorrichtung zum Separieren von Stammzellen gemäß einer ersten bevorzugten Ausführungsvariante. Dabei weist die Vorrichtung 100 einen Behälter 110 auf, welcher eine erste Kammer 171, eine, in einer Fließrichtung folgend, zweite Kammer 172, eine, in der Fließrichtung folgend, dritte Kammer 173 und eine, in der Fließrichtung folgend, vierte Kammer 174 umfasst. Die erste Kammer 171, die zweite Kammer 172 und die vierte Kammer 174 sind jeweils von einem Glaszylinder ummantelt. Die dritte Kammer 173 ist von einem Filterzylinder, auch Radialzylinder genannt, ummantelt.

Die erste Kammer 171 weist im Deckel eine Stoffgemischzuführeinrichtung 130 sowie eine Spülmittelzuführeinrichtung 120 auf, welche in dieser Darstellung beide in einer Öffnung des Deckels zusammentreffen. Weiterhin weist der Deckel ein erstes Druckausgleichventil 181 auf, welches sowohl einen Druckausgleich zwischen einem Äußeren und einem Inneren des Behälters als auch eine Austragung von Spülmittel, Enzymen und dem um Stammzellen reduzierten Stoffgemisch ermöglicht.

Die erste Kammer 171 weist weiterhin ein aktives Element, insbesondere einen Stempelkolben 121, auf, welcher vertikal beweglich ist. Der Stempelkolben 121 kann auch als Prallelement 340 bezeichnet werden, da durch dessen Bewegung das Fluid auf diesen prallt. Infolge der Bewegung und der räumlichen Ausdehnung des Stempelkolbens 121 wird das Fluid mit dem Stoffgemisch verdrängt und in Strömung versetzt. Der Stempelkolben 121 ist pneumatisch, beispielsweise mittels pulsierendem Unterdruck (Vakuum 190), zur Oszillation, also einer wiederholten Auf- und Ab-Bewegung, antreibbar, wobei eine rückführende Kraft mittels eines Federelementes zur Verfügung gestellt wird.

Am Boden der ersten Kammer 171 befindet sich eine erste spezifisch durchlässige Membran 161 in Form eines ersten Siebes in direktem Kontakt zum mit Fluid vermischten Stoffgemisch. Die erste Membran 161 weist eine Lochgröße von 100 µm auf. In Richtung der Schwerkraft folgt eine zweite spezifisch durchlässige Membran 162 (ein zweites Sieb) und eine dritte spezifisch durchlässige Membran 163 (ein drittes Sieb), gefolgt von einem Stützsieb.

Um ein Anhaften von Partikeln an den Membranen zu vermeiden, ist unterhalb der Membranen ein Vibrator 122 angeordnet und mit den Membranen schwingungsübertragend verbunden.

In Richtung der Schwerkraft folgt unterhalb der ersten Kammer 171 und unterhalb der ersten Membran 161 die zweite Kammer 172. Die beiden Kammern sind dabei strömungstechnisch miteinander verbunden, wobei die Verbindung ein erstes Ventil 131 aufweist, um den Zeitpunkt bestimmen zu können, zu welchem das Stoffgemisch von der ersten Kammer 171 in die zweite Kammer 172 fließt, oder anders ausgedrückt, wann die Membranen (Filter) öffnen oder schließen.

Die zweite Kammer 172 weist ebenfalls ein Druckausgleichventil, das zweite Druckausgleichventil 182, auf, welches sowohl einen Druckausgleich zwischen einem Äußeren und einem Inneren des Behälters als auch eine Austragung von Spülmittel, Enzymen und dem um Stammzellen reduzierten Stoffgemisch ermöglicht. Am Boden der zweiten Kammer 172 befindet sich wiederum eine Membran, die vierte spezifisch durchlässige Membran 164, also das vierte Sieb. Die vierte Membran 164 weist eine Lochgröße von 10 µm auf.

In Richtung der Schwerkraft folgt unterhalb der zweiten Kammer 172 und unterhalb der vierten Membran 164 die dritte Kammer 173. Die beiden Kammern sind dabei strömungstechnisch miteinander verbunden, wobei die Verbindung ein zweites Ventil 132 aufweist, um den Zeitpunkt bestimmen zu können, zu welchem das Stoffgemisch von der zweiten Kammer 172 in die dritte Kammer 173 fließt, oder anders ausgedrückt, wann die vierte Membran 164 (der vierte Filter) öffnet oder schließt.

Die dritte Kammer 173 ist seitlich von einer zylinderförmig angeordneten Membran, der fünften spezifisch durchlässigen Membran 165, auch Radialsieb-Zylinder, Radialzylinder oder Filterzylinder genannt, umgeben. Dabei weist die fünfte Membran 165 eine Lochgröße von 1 µm auf.

Die Stammzellen passen nicht durch diese Lochgröße. Daher sammeln sich die Stammzellen in einem zirka 5 ml großen Reservoir für Stammzellen am Boden der dritten Kammer 173, also am Boden innerhalb des Radialsieb-Zylinders, und können von dort mittels einer Stammzellenentnahmeeinrichtung 140, welche ein Ventil und einen Anschluss zu einer Spritze aufweist, abgesaugt werden.

Auch die vierte Kammer 174 weist ein Druckausgleichventil, das dritte Druckausgleichventil 183, auf, welches einen Druckausgleich zwischen einem Äußeren und einem Inneren des Behälters ermöglicht. Ebenso weist die vierte Kammer 174 eine Spülmittelabführeinrichtung 150 auf, die eine Austragung von Spülmittel, Enzymen und dem um Stammzellen reduzierten Stoffgemisch ermöglicht. Durch eine bei Bedarf wechselnde Saug- und Druckspülung an der Spülmittelabführeinrichtung 150 wird ebenfalls ein Anhaften der Stammzellen an der Radialfiltermembran verhindert.

Die dritte Kammer 173 enthält ein geschlossenes Volumenelement 175, welches für beispielsweise Reinigungszwecke der Kammer 173 entnehmbar ist. Das Volumenelement 175 umfasst in etwa 70 % bis 90 % des Volumens der Kammer 173.

Das Volumenelement 175 weist im dem zweiten Ventil 132 zugewandten Bereich eine Wölbung auf, so dass das zugeführte Stoffgemisch auf den höchsten Punkt des Volumenelements 175, die Wölbung, trifft, und von dort aus in Richtung der Seiten, also der Zylindermantelfläche des Volumenelements 175 hin abläuft.

Damit das Volumenelement 175 zentriert innerhalb der dritten Kammer 173 angeordnet bleibt, nicht die Öffnungen der fünften Membran 165 versperrt und dennoch genügend Platz für das zugeführte Stoffgemisch und auch Platz für die sich absetzenden, an der fünften Membran 165 in das Reservoir hinab gleitenden, Stammzellen bereit hält, weist das Volumenelement 175 im jeweils oberen und unteren Bereich eine nicht durchgehend umlaufende, radial auskragende Aufweitung auf. Nicht durchgehend umlaufend bedeutet, dass die Auskragung keinen geschlossenen Ring um das Volumenelement 175 ausbildet, sondern dass der Ring an seinem äußeren Rand Einkerbungen oder Aussparungen, insbesondere Zinken, aufweist, also äußerlich gezinkt ist. Die Zinken können dabei verschiedenartig geformt sein, beispielsweise ähnlich der Form einer Fächerrosette. Vorteilhafterweise befindet sich das Volumenelement 175 dadurch nahezu wackel- und klapperfrei innerhalb der dritten Kammer 173.

Damit die fünfte Membran 165 durch die wechselnde Saug- und Druckspülung über die Spülmittelabführeinrichtung 150 nicht beschädigt wird, ist die fünfte Membran 165 durch ein Filtergehäuse 166 in Saugrichtung abgestützt.

Bei Druckumkehr/Druckspülung ist das Spaltmaß zwischen der fünften Membran 165 und dem Volumenelement 175 derart bemessen, dass sich die fünfte Membran 165 am Volumenelement 175 abstützt.

Das Filtergehäuse 166 weist radiale Schlitze 167 auf und das geschlossene Volumenelement 175 weist radiale, nicht durchgehende Aufweitungen, für eine optimale Durchlässigkeit der Membran 165, wenn diese bei der Saug-/Druckspülung abgestützt wird, auf.

Der Behälter 110 ist zwecks einer besseren Reinigung weitgehend zerlegbar.

Damit der Behälter 110 nicht kippelt, weist er einen Fuß auf, der gegenüber der übrigen zylinderähnlichen Form des Behälters 110 einen größeren Durchmesser aufweist als der filterfunktionale Teil des Behälters 110.

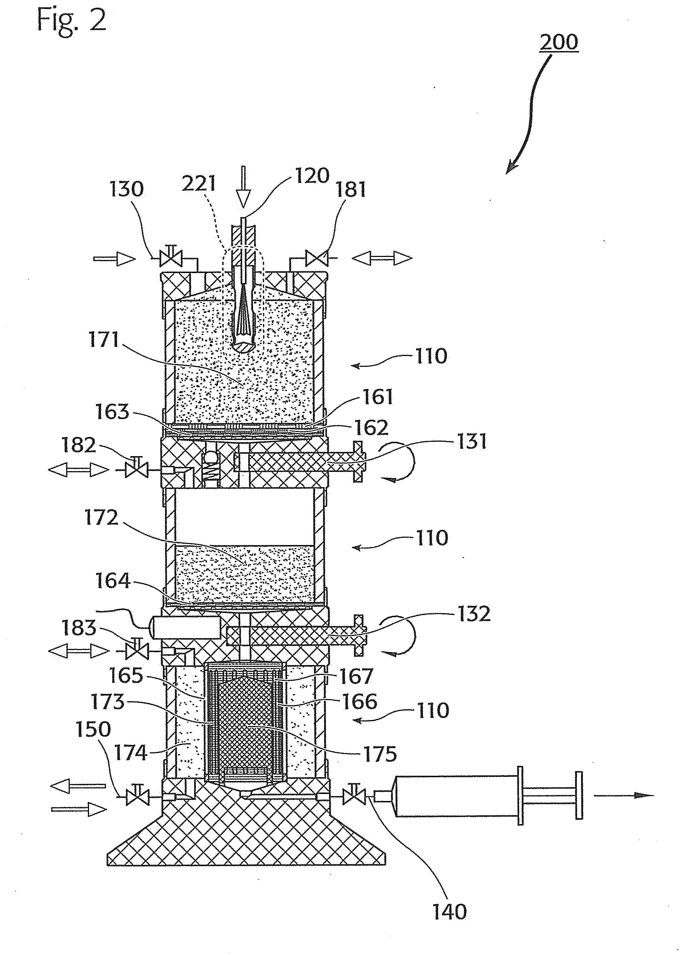

Figur 2 zeigt eine zweidimensionale schematische Darstellung einer insgesamt mit 200 bezeichneten Vorrichtung zum Separieren von Stammzellen gemäß einer zweiten bevorzugten Ausführungsvariante.

Dabei ist die im Deckel befindliche Stoffgemischzuführeinrichtung 130 von der im Deckel befindlichen Spülmittelzuführeinrichtung 120 räumlich getrennt und beabstandet. Die Spülmittelzuführeinrichtung 120 ist mit dem aktiven Element, einer Venturi-Düse 221, kombiniert, welche in die erste Kammer 171 mündet.

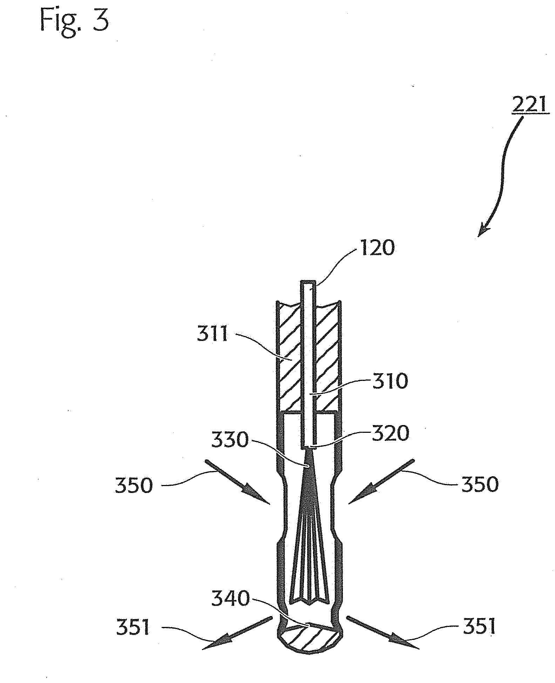

Figur 3 zeigt eine zweidimensionale, vergrößerte schematische Darstellung der insgesamt mit 221 bezeichneten Venturi-Düse als aktives Element. Dabei weist die Venturi-Düse 221 ein Kanülenrohr 311, welches eine Düsenkapillare 310 in sich führt, auf. Die Düsenkapillare 310 weist in Strahlrichtung eine Austrittsöffnung 320 auf, durch welche ein Fluid 330, insbesondere das Spülmittel, mit einem erhöhten Druck versehen, in die erste Kammer 171 strömbar ist. Durch die Fließgeschwindigkeit des Fluids 330 wird das Stoffgemisch, auch als Gewebe 350 bezeichnet, angesaugt und mit dem Strahl mitgerissen. Der mitreißende Strahl strömt auf ein Prallelement 340. Dadurch wird das Gewebe 350 mit dem mit Enzymen versetzten Spülmittel versetzt und vermischt und gelangt als Gewebeemulsion 351, also besser vermischt, aus der Venturi-Düse 221 in die erste Kammer 171.

### Bezugszeichen liste

- 100: erste Vorrichtung zum Separieren von adulten Stammzellen
- 110: Behälter zur Aufnahme eines Stoffgemischs
- 120: Spülmittelzuführeinrichtung
- 121: Stempelkolben als aktives Element
- 122: Vibrator
- 130: Stoffgemischzuführeinrichtung
- 131: erstes Ventil
- 132: zweites Ventil
- 140: Stammzellenentnahmeeinrichtung
- 150: Spülmittelabführeinrichtung
- 161: erste spezifisch durchlässige Membran
- 162: zweite spezifisch durchlässige Membran
- 163: dritte spezifisch durchlässige Membran
- 164: vierte spezifisch durchlässige Membran
- 165: fünfte spezifisch durchlässige Membran
- 166: Filtergehäuse
- 167: radiale Schlitze
- 171: erste Kammer
- 172: zweite Kammer
- 173: dritte Kammer
- 174: vierte Kammer
- 175: Volumenelement
- 181: erstes Druckausgleichventil
- 182: zweites Druckausgleichventil
- 183: drittes Druckausgleichventil
- 190: Vakuum
- 200: zweite Vorrichtung zum Separieren von adulten Stammzellen
- 221: Venturi-Düse als aktives Element
- 310: Düsenkapillare
- 311: Kanülenrohr
- 320: Austrittsöffnung
- 330: Fluid
- 340: Prallelement
- 350: angesaugtes Gewebe
- 351: Gewebeemulsion

## Patentansprüche

1. Vorrichtung (100, 200) zum Separieren von adulten Stammzellen aus einem einer biologischen Struktur entnommenen Fettgewebe, aufweisend:
- einen Behälter (110) zur Aufnahme eines Stoffgemischs, welches das Fettgewebe und die adulten Stammzellen umfasst und der wenigstens drei Kammern aufweist (171 -174);
- wenigstens eine Spülmittelzuführeinrichtung (120), die ausgebildet ist, ein Fluid (330) in den Behälter (110) einzuleiten;
- eine Stoffgemischzuführeinrichtung (130), die ausgebildet ist, das Stoffgemisch in den Behälter (110) einzuleiten;
- eine Stammzellenentnahmeeinrichtung (140), die ausgebildet ist, die separierten Stammzellen dem Behälter (110) zu entnehmen;
- wenigstens eine Spülmittelabführeinrichtung (150), die ausgebildet ist, ein um adulte Stammzellen reduziertes Stoffgemisch aus dem Behälter (110) abzuführen und
- wenigstens zwei spezifisch durchlässige Membranen (161-165), die für jeweils nur einen spezifischen Teil des jeweiligen Stoffgemischs durchlässig sind,
wobei
- die wenigstens drei Kammern (171 - 174) wenigstens eine erste Kammer (171), eine dritte Kammer (173) und eine vierte Kammer (174) umfassen,
- von denen die erste Kammer (171) strömungstechnisch entweder direkt mit der dritten Kammer (173) oder mit einer zweiten Kammer (172) verbunden ist, wobei im letzten Fall die zweite Kammer wiederum strömungstechnisch direkt mit der dritten Kammer (173) verbunden ist und
- von denen die dritte Kammer (173) strömungstechnisch direkt mit der vierten Kammer (174) verbunden ist, wobei
- die erste Kammer (171) von der dritten Kammer (173) durch wenigstens eine Membran (161-164) getrennt ist, die für die adulten Stammzellen durchlässig ist und
- die dritte Kammer (173) von der vierten Kammer (174) durch eine Membran (165) getrennt ist, die undurchlässig für die adulten Stammzellen ist und weiterhin
- die erste Kammer (171) mit der Stoffgemischzuführeinrichtung (130) und der Spülmittelzuführeinrichtung (120) verbunden ist,
- die dritte Kammer (173) mit der Stammzellenentnahmeeinrichtung (140) verbunden ist und
- die vierte Kammer (174) mit der Spülmittelabführeinrichtung (150) verbunden ist
**dadurch gekennzeichnet, dass**
die Membran (165), die die dritte Kammer (173) von der vierten Kammer (174) trennt, als Radialfilter ausgebildete ist und die dritte Kammer (173) seitlich umgibt.

2. Vorrichtung nach Anspruch 1, wobei die wenigstens drei Kammern (171-174) wenigstens ein Druckausgleichventil (181-183) aufweisen, welches ausgebildet ist,
- einen Druck im Inneren des Behälters (110) gegenüber einem Druck außerhalb des Behälters (110) auszugleichen und/oder
- wiederholbar geöffnet und geschlossen zu werden.

3. Vorrichtung nach einem der vorhergehenden Ansprüche, wobei wenigstens eine von den wenigstens drei Kammern (171-174) ein mechanisch aktives Element (121, 221) aufweist, welches ausgebildet ist, das Fluid (330) mit dem Stoffgemisch zu vermischen.

4. Vorrichtung nach Anspruch 3, **wobei** das aktive Element (121, 221) vom Fluid (330) durchströmbar und weiterhin ausgebildet ist, dass ein dynamischer Unterdruck infolge einer Fließgeschwindigkeit des Fluids (330) das Stoffgemisch an das Fluid (330) ansaugt und das Stoffgemisch in Richtung der Fließgeschwindigkeit des Fluids (330) beschleunigt wird.

5. Vorrichtung nach Anspruch 4, **wobei** das aktive Element (121, 221) ein Prallelement (340) aufweist, welches derart angeordnet ist, dass das beschleunigte Stoffgemisch mit dem strömenden Fluid (330) auf das Prallelement (340) trifft.

6. Vorrichtung nach Anspruch 3, **wobei** das aktive Element (121, 221) ein Rührpendel aufweist, welches ausgebildet ist, eine quirlartige Drehbewegung und/oder eine schaukelartige Pendelbewegung auszuführen.

7. Vorrichtung nach Anspruch 3, **wobei** das aktive Element (121, 221) einen ortsveränderlichen Stempelkolben (121) aufweist, der ausgebildet ist, infolge seiner räumlichen Ausdehnung das Fluid (330) mit dem Stoffgemisch zu verdrängen und wiederholbar seinen Ort der Verdrängung zu verändern.

8. Vorrichtung nach Anspruch 7, **wobei** der Stempelkolben (121) mittels Vakuum (190) zur Oszillation antreibbar ist.

9. Vorrichtung nach einem der vorhergehenden Ansprüche, **wobei** der Behälter (110) einen Vibrator (122) aufweist, welcher ausgebildet ist, das Fluid (330) mit dem Stoffgemisch in Schwingung zu versetzen.

10. Vorrichtung nach einem der vorhergehenden Ansprüche, **wobei** die Verbindung zwischen den jeweiligen strömungstechnisch direkt miteinander verbundenen Kammern (171-174) jeweils ein Ventil (131, 132) aufweist, welches ausgebildet ist, wiederholbar geöffnet und geschlossen zu werden.

11. Vorrichtung nach einem der vorhergehenden Ansprüche, **wobei** die Spülmittelabführeinrichtung (150) ausgebildet ist, eine wechselnde Saug- und Druckspülung zu gewährleisten.

12. Vorrichtung nach einem der vorhergehenden Ansprüche, **wobei** der Behälter (110) ein Temperierelement aufweist, welches ausgebildet ist, das Fluid (330) und/oder das Stoffgemisch zu kühlen und/oder zu erwärmen.

13. Vorrichtung nach einem der vorhergehenden Ansprüche, **wobei** die wenigstens eine Membran (165) eine elektrostatische Polarisierung aufweist.

14. Vorrichtung nach einem der vorhergehenden Ansprüche, **wobei** die dritte Kammer (173) ein geschlossenes Volumenelement (175) aufweist, dessen Volumen eine Größe von mindestens 50 % eines Volumens der dritten Kammer (173) aufweist.

## Claims

1. A device (100, 200) for separating adult stem cells from adipose tissue taken from a biological structure, having:
- a container (110) for receiving a material mixture comprising the adipose tissue and the adult stem cells and which has at least three chambers (171-174);
- at least one rinse agent feed apparatus (120) configured to introduce a fluid (330) into the container (110);
- a material mixture feed apparatus (130) configured to introduce the material mixture into the container (110);
- a stem cell extraction apparatus (140) configured to remove the separated stem cells from the container (110);
- at least one rinse agent drainage apparatus (150) configured to discharge a material mixture reduced in terms of adult stem cells from the container (110), and
- at least two specifically permeable membranes (161-165) which are each permeable to only a specific part of the respective material mixture,
wherein
- the at least three chambers (171-174) comprise at least a first chamber (171), a third chamber (173) and a fourth chamber (174),
- the first chamber (171) of which is either directly connected to the third chamber (173) or to a second chamber (172) for fluid communication, wherein in the latter case, the second chamber is in turn directly connected to the third chamber (173) for fluid communication, and
- the third chamber (173) of which is directly connected to the fourth chamber (174) for fluid communication, wherein
- the first chamber (171) is separated from the third chamber (173) by at least one membrane (161-164) which is permeable to the adult stem cells, and
- the third chamber (173) is separated from the fourth chamber (174) by a membrane (165) which is impermeable to the adult stem cells, and furthermore
- the first chamber (171) is connected to the material mixture feed apparatus (130) and the rinse agent feed apparatus (120),
- the third chamber (173) is connected to the stem cell extraction apparatus (140), and
- the fourth chamber (174) is connected to the rinse agent drainage apparatus (150),
**characterized in that**
the membrane (165) which separates the third chamber (173) from the fourth chamber (174) is configured as a radial filter and surrounds the third chamber (173) laterally.

2. The device according to Claim 1, **wherein** the at least three chambers (171-174) have at least one pressure equalization valve (181-183) which is configured
- to equalize a pressure in the interior of the container (110) relative to a pressure outside the container (110), and/or
- to be repeatedly opened and closed.

3. The device according to any one of the preceding claims, **wherein** at least one of the at least three chambers (171-174) has a mechanically active element (121, 221) which is configured to mix the fluid (330) with the material mixture.

4. The device according to Claim 3, **wherein** the fluid (330) can flow through the active element (121, 221) and wherein the active element (121, 221) is furthermore configured so that a dynamic negative pressure caused by a flow rate of the fluid (330) sucks the material mixture towards the fluid (330), and the material mixture is accelerated in the direction of the flow velocity of the fluid (330).

5. The device according to Claim 4, **wherein** the active element (121, 221) has an impact element (340) which is arranged such that the accelerated material mixture with the flowing fluid (330) strikes the impact element (340).

6. The device according to Claim 3, **wherein** the active element (121, 221) has a stirring pendulum which is configured to perform a stirring rotational movement and/or a swing-type pendulum motion.

7. The device according to Claim 3, **wherein** the active element (121, 221) has a non-stationary stamp piston (121) which is configured to displace the fluid (330) having the material mixture due to its spatial extent and to repeatedly change its location of displacement.

8. The device according to Claim 7, **wherein** the stamp piston (121) can be driven to oscillate by means of a vacuum (190).

9. The device according to any one of the preceding claims, **wherein** the container (110) has a vibrator (122) which is configured to introduce oscillations in the fluid (330) having the material mixture.

10. The device according to any one of the preceding claims, **wherein** the connection between the respective chambers (171-174) which are directly connected to one another for fluid communication has, in each case, a valve (131, 132) which is configured to be repeatedly opened and closed.

11. The device according to any one of the preceding claims, **wherein** the rinse agent drainage apparatus (150) is configured to ensure an alternating suction and pressurized rinse.

12. The device according to any one of the preceding claims, **wherein** the container (110) has a temperature-control element which is configured to cool and/or heat the fluid (330) and/or material mixture.

13. The device according to any one of the preceding claims, **wherein** the at least one membrane (165) has an electrostatic polarization.

14. The device according to any one of the preceding claims, **wherein** the third chamber (173) has a closed volume element (175), the volume of which is at least 50% of a volume of the third chamber (173).

## Revendications

1. Dispositif (100, 200) pour la séparation de cellules souches adultes à partir d'un tissu graisseux prélevé sur une structure biologique, comprenant :
- un contenant (110) pour la réception d'un mélange de matières, qui comprend le tissu graisseux et les cellules souches adultes, et qui comprend au moins trois chambres (171-174);
- au moins un dispositif d'introduction d'un agent de rinçage (120), qui est configuré pour introduire un fluide (330) dans le contenant (110) ;
- un dispositif d'introduction du mélange de matières (130), qui est configuré pour introduire le mélange de matières dans le contenant (110) ;
- un dispositif d'extraction de cellules souches (140), qui est configuré pour extraire les cellules souches séparées du contenant (110) ;
- au moins un dispositif de déchargement de l'agent de rinçage (150), qui est configuré pour décharger un mélange de matières à teneur réduite en cellules souches adultes du contenant (110), et
- au moins deux membranes à perméabilité spécifique (161-165), qui sont chacune perméables uniquement à une partie spécifique du mélange de matières en question,
- lesdites au moins trois chambres (171-174) comprenant au moins une première chambre (171), une troisième chambre (173) et une quatrième chambre (174),
- parmi lesquelles la première chambre (171) est raccordée en termes d'écoulement soit directement avec la troisième chambre (173), soit avec une deuxième chambre (172) ; dans le dernier cas, la deuxième chambre étant elle-même raccordée en termes d'écoulement directement avec la troisième chambre (173), et
- parmi lesquelles la troisième chambre (173) est raccordée directement en termes d'écoulement avec la quatrième chambre (174),
- la première chambre (171) étant séparée de la troisième chambre (173) par au moins une membrane (161-164), qui est perméable aux cellules souches adultes, et
- la troisième chambre (173) étant séparée de la quatrième chambre (174) par une membrane (165), qui est imperméable aux cellules souches adultes, et
- la première chambre (171) étant en outre raccordée avec le dispositif d'introduction du mélange de matières (130) et le dispositif d'introduction de l'agent de rinçage (120),
- la troisième chambre (173) étant raccordée avec le dispositif d'extraction de cellules souches (140), et
- la quatrième chambre (174) étant raccordée avec le dispositif de déchargement de l'agent de rinçage (150),
**caractérisé en ce que**
la membrane (165) qui sépare la troisième chambre (173) de la quatrième chambre (174) est configurée sous la forme d'un filtre radial, et entoure latéralement la troisième chambre (173).

2. Dispositif selon la revendication 1, **dans lequel** lesdites au moins trois chambres (171-174) comprennent au moins une soupape d'égalisation de la pression (181-183), qui est configurée
- pour égaliser une pression à l'intérieur du contenant (110) par rapport à une pression à l'extérieur du contenant (110) et/ou
- pour être ouverte et fermée de manière répétée.

3. Dispositif selon l'une quelconque des revendications précédentes, **dans lequel** au moins une desdites au moins trois chambres (171-174) comprend un élément mécaniquement actif (121, 221), qui est configuré pour mélanger le fluide (330) avec le mélange de matières.

4. Dispositif selon la revendication 3, **dans lequel** l'élément actif (121, 221) peut être traversé par le fluide (330) et est en outre configuré de telle sorte qu'une sous-pression dynamique résultant d'une vitesse d'écoulement du fluide (330) aspire le mélange de matières vers le fluide (330) et accélère le mélange de matières dans la direction de la vitesse d'écoulement du fluide (330).

5. Dispositif selon la revendication 4, **dans lequel** l'élément actif (121, 221) comprend un élément d'impact (340), qui est agencé de telle sorte que le mélange de matières accéléré rencontre le fluide en écoulement (330) sur l'élément d'impact (340).

6. Dispositif selon la revendication 3, **dans lequel** l'élément actif (121, 221) comprend un pendule d'agitation, qui est configuré pour réaliser un mouvement de rotation en fouet et/ou un mouvement de pendule en balancement.

7. Dispositif selon la revendication 3, **dans lequel** l'élément actif (121, 221) comprend un piston de poinçon non stationnaire (121), qui est configuré pour refouler le fluide (330) avec le mélange de matières à la suite de son expansion spatiale, et modifier de manière répétée son emplacement de refoulement.

8. Dispositif selon la revendication 7, **dans lequel** le piston de poinçon (121) est mis en oscillation par un vide (190).

9. Dispositif selon l'une quelconque des revendications précédentes, dans lequel le contenant (110) comprend un vibrateur (122), qui est configuré pour mettre en vibration le fluide (330) avec le mélange de matières.

10. Dispositif selon l'une quelconque des revendications précédentes, **dans lequel** la liaison entre les chambres (171-174) raccordées directement les unes avec les autres en termes d'écoulement comprend à chaque fois une soupape (131, 132), qui est configurée pour être ouverte et fermée de manière répétée.

11. Dispositif selon l'une quelconque des revendications précédentes, **dans lequel** le dispositif de déchargement de l'agent de rinçage (150) est configuré pour assurer un rinçage par aspiration et compression en alternance.

12. Dispositif selon l'une quelconque des revendications précédentes, **dans lequel** le contenant (110) comprend un élément de conditionnement en température, qui est configuré pour refroidir et/ou chauffer le fluide (330) et/ou le mélange de matières.

13. Dispositif selon l'une quelconque des revendications précédentes, **dans lequel** ladite au moins une membrane (165) comprend une polarisation électrostatique.

14. Dispositif selon l'une quelconque des revendications précédentes, **dans lequel** la troisième chambre (173) comprend un élément de volume fermé (175), dont le volume présente une taille d'au moins 50 % d'un volume de la troisième chambre (173).
